# EUROPEAN PATENT APPLICATION

(11) **EP 4 566 467 A1**
(43) Date of publication of application: **11.06.2025**
(21) Application number: 24160577.3
(22) Date of filing: 29.02.2024
(51) Int. Cl.: A24F 40/40, H01G 11/08, H01G 11/50, H01G 11/36, H01G 11/62, H01G 11/06, A24F 40/50, A24F 40/90, A61M 15/06, H01G 11/38, H01G 11/60

(54) **AEROSOL PROVISION DEVICE**

(30) Priority: 08.12.2023 GB 202318766; 08.12.2023 GB 202318765; 08.12.2023 GB 202318763; 08.12.2023 GB 202318768; 08.12.2023 GB 202318767
(71) Applicant: Nicoventures Trading Limited, London WC2R 3LA (GB)
(72) Inventor: HU, Yu, London, WC2R 3LA (GB); TAN, Chaou Choak, London, WC2R 3LA (GB); BRUTON, Connor, London, WC2R 3LA (GB); POYNTON, Simon, London, WC2R 3LA (GB)
(74) Representative: Cork, Robert

(57) **Abstract**

An aerosol provision device for providing an aerosol during a puff on the aerosol provision device is disclosed. The aerosol provision device comprises at least one power source comprising at least one supercapacitor configured to supply electrical power to at least one aerosol generator for generating the aerosol from aerosolgenerating material. The at least one supercapacitor is a lithium-ion capacitor, LIC, comprising an anode and a cathode, wherein said anode and/or said cathode comprises one or more dopants.

## Description

### Technical Field

The present invention relates to an aerosol provision device.

### Background

Electronic aerosol provision systems such as electronic cigarettes (e-cigarettes) generally contain an aerosol-generating material, such as a reservoir of a source liquid containing a formulation, typically including nicotine, or a solid material such as a tobacco-based product, from which an aerosol is generated for inhalation by a user, for example through heat vaporisation. Thus, an aerosol provision system will typically comprise an aerosol generator, e.g. a heating element, arranged to aerosolise a portion of aerosol-generating material to generate an aerosol in an aerosol generation region of an air channel through the aerosol provision system. As a user inhales on the device and electrical power is supplied to the aerosol generator, air is drawn into the device through one or more inlet holes and along the air channel to the aerosol generation region, where the air mixes with the vaporised aerosol generator and forms a condensation aerosol. The air drawn through the aerosol generation region continues along the air channel to a mouthpiece, carrying some of the aerosol with it, and out through the mouthpiece for inhalation by the user.

It is common for aerosol provision systems to comprise a rechargeable power source, for example a battery such as a lithium-ion battery, which can supply power to the aerosol generator to generate the aerosol. The rechargeable power source can be charged from an external power source, for example the mains power supply. The rechargeable power source can allow the device to be recharged and re-used many times over, in contrast with so-called 'disposable' devices which can only be used once and must be disposed of and replaced once the energy in their internal battery has been depleted. However, rechargeable power sources are hampered by drawbacks of existing battery technologies, such as relative slow charging times, low instantaneous charging/discharging current and a limited lifespan (i.e. in terms of the total number of charge/discharge cycles that can be achieved). It would therefore be desirable to provide an aerosol provision system with an improved rechargeable power source.

### Summary of the Invention

According to a first aspect of the present disclosure, there is provided an aerosol provision device for providing an aerosol during a puff on the aerosol provision device, the aerosol provision device comprising: at least one power source comprising at least one supercapacitor configured to supply electrical power to at least one aerosol generator for generating the aerosol from aerosol-generating material, wherein the at least one supercapacitor is a lithium-ion capacitor, LIC, comprising an anode and a cathode, and wherein said anode and/or said cathode comprises one or more dopants.

In some embodiments according to the first aspect, said cathode comprises one or more of: activated carbon, heteroatom-doped carbon, or graphene.

In some embodiments according to the first aspect, said cathode comprises one or more of the following as said one or more dopants: lithium cobalt oxide, LCO; lithium manganese oxide, LMO; lithium iron phosphate, LFP; lithium iron manganese phosphate, LMFP; lithium nickel aluminium oxide, NCA; and lithium nickel manganese cobalt oxide, NMC.

In some embodiments according to the first aspect, said anode comprises one or more of: lithium pre-doped graphitic carbon; hard carbon and/or soft carbon; lithium titanium oxide, LTO; and iron oxide, Fe₂O₃.

In some embodiments according to the first aspect, said one or more dopants include one or more cation dopants.

In some embodiments according to the first aspect, said one or more cation dopants comprise one or more of: Na; K; Mg; Zn; Ca; Fe; Al; Y; Cr; Mo; Te; Zr; Si; Sn; Ti; Ru; V; W; Ta; Ga; and Nb.

In some embodiments according to the first aspect, said one or more dopants include one or more anion dopants.

In some embodiments according to the first aspect, said one or more anion dopants comprise one or more of: F; Cl; S; N; Si; B; and P.

In some embodiments according to the first aspect, the at least one supercapacitor comprises a liquid electrolyte.

In some embodiments according to the first aspect, the liquid electrolyte comprises one or more of the following: a lithium salt; lithium hexafluoroarsenate, LiAsF₆; lithium bis (fluorosulfonyl) imide, LiFSI; lithium bis (trifluoromethanesulfonyl) imide, LiTFSI; lithium tetrafluoroborate, LiBF₄; lithium perchlorate, LiClO₄; and lithium bis(oxalato) borate, LiBOB.

In some embodiments according to the first aspect, the lithium salt comprises lithium hexafluorophosphate, LiPF₆.

In some embodiments according to the first aspect, the liquid electrolyte comprises a carbonate-based organic solvent.

In some embodiments according to the first aspect, the carbonate-based organic solvent comprises one or more of: ethylene carbonate, EC; propylene carbonate, PC; ethyl methyl carbonate, EMC; diethyl carbonate, DEC; dimethyl carbonate, DMC; propyl acetate, PA; methyl formate, MF; methyl acetate, MA; ethyl acetate, EA; methyl pyrrolidone, MP; tetrahydrofuran, THF; dimethylsulfoxide, DMSO; 1,2-dimenthoxyethane, DME; and dichloromethane, DCM.

In some embodiments according to the first aspect, the liquid electrolyte comprises a nitrile-based organic solvent.

In some embodiments according to the first aspect, the nitrile-based organic solvent comprises acetonitrile and/or acrylonitrile.

In some embodiments according to the first aspect, the liquid electrolyte comprises one or more additives.

In some embodiments according to the first aspect, said one or more additives comprise one or more of: vinylene carbonate, VC; vinyl ethylene carbonate, VEC; and fluoroethylene carbonate, FEC.

In some embodiments according to the first aspect, the at least one supercapacitor is capable of being charged from a discharged state to a charged state in a charging time of less than or equal to 12 minutes, and in the charged state the at least one supercapacitor is configured to store sufficient electrical charge to supply electrical power to the at least one aerosol generator for no more than 200 puffs.

In some embodiments according to the first aspect, between a first output voltage limit and a second output voltage limit, the at least one supercapacitor has a discharge capacity of no more than 450 mAh and/or has a charge capacity of no more than 400 mAh.

In some embodiments according to the first aspect, the at least one supercapacitor is configured to be operable for a number of charge and discharge cycles greater than or equal to 10,000 cycles, and wherein after said number of charge and discharge cycles the at least one supercapacitor is still capable of storing sufficient electrical charge to supply electrical power to the at least one aerosol generator for no more than 200 puffs.

In some embodiments according to the first aspect, the aerosol provision device comprises: circuitry configured to control a supply of electrical power to the at least one supercapacitor during a charging operation, to charge the at least one supercapacitor from a discharged state to a charged state, wherein the circuitry is configured to control the supply of electrical power to the at least one supercapacitor during the charging operation so as to charge the at least one supercapacitor at a C-rate greater than or equal to 25C, wherein between a first output voltage limit and a second output voltage limit, the at least one supercapacitor has a discharge capacity of no more than 450 mAh and/or has a charge capacity of no more than 400 mAh, and/or wherein in the charged state the at least one supercapacitor is configured to store sufficient electrical charge to supply electrical power to the at least one aerosol generator for no more than 200 puffs.

In some embodiments according to the first aspect, the at least one supercapacitor is in the form of a cell having any one of the following cell shapes: a cylindrical cell shape, a pouch cell shape, a rectangular cell shape, and a prismatic cell shape.

In some embodiments according to the first aspect, the at least one supercapacitor is configured to be removeable and/or replaceable from the aerosol provision device.

According to a second aspect of the present disclosure, there is provided an aerosol provision system comprising: a consumable; and the aerosol provision device according to the first aspect, wherein the aerosol provision device is configured to be releasably engageable with said consumable, wherein said consumable comprises the aerosol-generating material.

In some embodiments according to the second aspect, the consumable comprises the at least one aerosol generator.

### Brief Description of the Drawings

Embodiments of the present invention will now be described, by way of example only, with reference to the accompanying drawings, in which:
Figure 1 is a block diagram of a rechargeable non-combustible aerosol provision device, according to an example embodiment;
Figure 2 is a block diagram of a system comprising the rechargeable non-combustible aerosol provision device of Fig. 1 and an apparatus for recharging the aerosol provision device, according to an example embodiment;
Figure 3 illustrates an exploded view of a rechargeable non-combustible aerosol provision device comprising a Li-ion capacitor power source and a removable consumable part, according to an example embodiment;
Figure 4 schematically illustrates an electrode structure of a hybrid supercapacitor power source, according to an example embodiment;
Figure 5 is a table illustrating cell properties and test results for six different types of cells, including LIC hybrid supercapacitor cells according to example embodiments;
Figure 6 is a graph plotting the charging time versus the number of charging cycles for the six cells listed in Fig. 5;
Figure 7 is a graph plotting the charge capacity versus the number of charging cycles for the six cells listed in Fig. 5;
Figure 8 is a graph plotting the total puff count achieved from a full charge versus the number of charging cycles for the six cells listed in Fig. 5;
Figure 9 is a table listing charging rates (C-rates) that have been demonstrated for each of the six cells listed in Fig. 5; and
Figure 10 schematically illustrates an aerosol provision device comprising a hybrid supercapacitor power source, according to an example embodiment.

### Detailed Description

In the following detailed description, only certain exemplary embodiments of the present invention have been shown and described, simply by way of illustration. As those skilled in the art would realise, the described embodiments may be modified in various ways, all without departing from the scope of the present invention. Accordingly, the drawings and description are to be regarded as illustrative in nature and not restrictive. Like reference numerals designate like elements throughout the specification.

As used herein, the term "delivery system" is intended to encompass systems that deliver at least one substance to a user, and includes non-combustible aerosol provision systems that release compounds from an aerosol-generating material without combusting the aerosol-generating material, such as electronic cigarettes, tobacco heating products, and hybrid systems to generate aerosol using a combination of aerosol-generating materials.

According to the present disclosure, a "combustible" aerosol provision system is one where a constituent aerosol-generating material of the aerosol provision system (or component thereof) is combusted or burned during use in order to facilitate delivery of at least one substance to a user. According to the present disclosure, a "non-combustible" aerosol provision system is one where a constituent aerosol-generating material of the aerosol provision system (or component thereof) is not combusted or burned in order to facilitate delivery of at least one substance to a user. The non-combustible aerosol provision system, or a non-combustible aerosol provision device thereof, may comprise a power source and a controller.

In some embodiments the delivery system is a non-combustible aerosol provision system, such as an electronic cigarette, also known as a vaping device or electronic nicotine delivery system (END), although it is noted that the presence of nicotine in the aerosol generating material is not a requirement. In some embodiments, the non-combustible aerosol provision system is an aerosol-generating material heating system, also known as a heat-not-burn system. An example of such a system is a tobacco heating system. In some embodiments, the non-combustible aerosol provision system is a hybrid system to generate aerosol using a combination of aerosol-generating materials, one or a plurality of which may be heated. Each of the aerosol-generating materials may be, for example, in the form of a solid, liquid or gel and may or may not contain nicotine. In some embodiments, the hybrid system comprises a liquid or gel aerosol-generating material and a solid aerosol-generating material. The solid aerosol-generating material may comprise, for example, tobacco or a non-tobacco product.

Typically, the non-combustible aerosol provision system may comprise a non-combustible aerosol provision device and a consumable for use with the non-combustible aerosol provision device. A non-combustible aerosol provision device may also be referred to as a non-combustible aerosol generation device. In some embodiments, the disclosure relates to consumables comprising aerosol generating material and configured to be used with non-combustible aerosol provision devices. These consumables are sometimes referred to as articles throughout the disclosure. A consumable is an article comprising or consisting of aerosol-generating material, part or all of which is intended to be consumed during use by a user.

In some embodiments, the non-combustible aerosol provision system may comprise an area for receiving the consumable, an aerosol generator, an aerosol generation area, a housing, a mouthpiece, a filter and/or an aerosol-modifying agent.

In some embodiments, the consumable for use with the non-combustible aerosol provision device may comprise aerosol-generating material, an aerosol-generating material storage area, an aerosol-generating material transfer component, an aerosol generator, an aerosol generation area, a housing, a wrapper, a filter, a mouthpiece, and/or an aerosol-modifying agent. A consumable may also comprise an aerosol generator, such as a heater, that emits heat to cause the aerosol-generating material to generate aerosol in use. The heater may, for example, comprise combustible material, a material heatable by electrical conduction, or a susceptor. In other embodiments, the aerosol-generating material may be provided in the non-combustible aerosol provision device itself rather than being provided in a physically separate consumable. In such embodiments, the non-combustible aerosol provision device may comprise an aerosol-generating material storage area for storing the aerosol-generating material.

In some embodiments, the substance to be delivered may be an aerosol-generating material or a material that is not intended to be aerosolised. As appropriate, either material may comprise one or more active constituents, one or more flavours, one or more aerosol-former materials, and/or one or more other functional materials.

In some embodiments, the substance to be delivered comprises an active substance. The active substance as used herein may be a physiologically active material, which is a material intended to achieve or enhance a physiological response. Aerosol-generating material is a material that is capable of generating aerosol, for example when heated, irradiated or energized in any other way. Aerosol-generating material may, for example, be in the form of a solid, liquid, gel, thin film, foam, or non-fibrous solid, any of which may or may not contain an active substance and/or flavourants.

The aerosol-generating material may comprise one or more active substances and/or flavours, one or more aerosol-former materials, and optionally one or more other functional materials.

The material may be present on or in a support, to form a substrate. The support may, for example, be or comprise paper, card, paperboard, cardboard, reconstituted material, a plastics material, a ceramic material, a composite material, glass, a metal, or a metal alloy. In some embodiments, the support comprises a susceptor. In some embodiments, the susceptor is embedded within the material. In some alternative embodiments, the susceptor is on one or either side of the material.

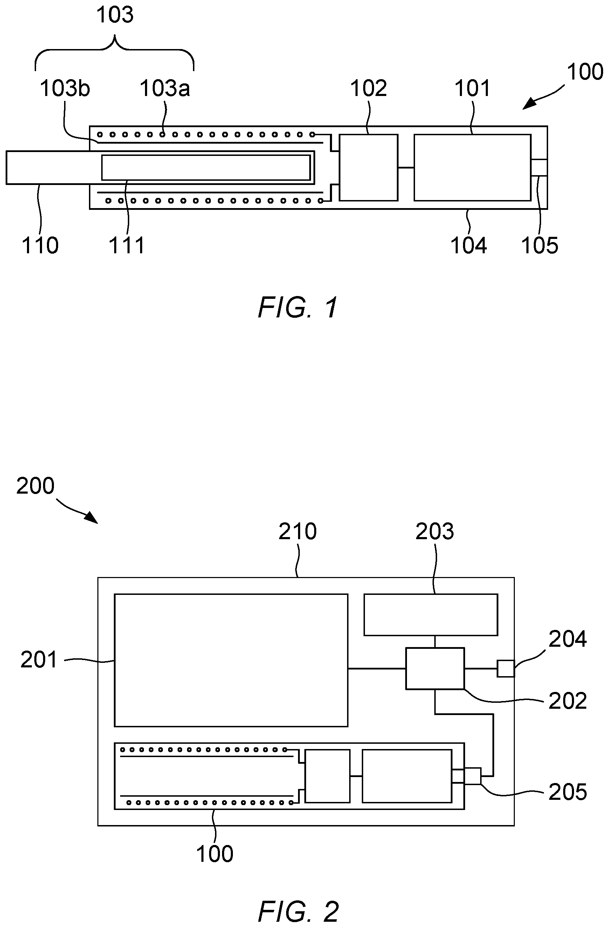

Referring now to Fig. 1, a block diagram of a rechargeable non-combustible aerosol provision device, indicated generally by the reference numeral 100, is illustrated in accordance with an example embodiment. The non-combustible aerosol provision device 100 comprises at least one power source 101, circuitry 102, at least one aerosol generator 103, a housing 104 and a charging interface 105.

The circuitry 102 may be configured to control a supply of electrical power to the at least one power source 101 during a charging operation, to charge the at least one power source 101 from a discharged state to a charged state (e.g. 80%, 90% or 100% charge). The discharged state may vary according to usage of the device 100, depending on how far the power source 101 has been discharged when a user connects the charging interface 105 to an external power source to begin charging the device 100. In general, the discharged state may be any state in which the output voltage of the power source 101 is greater than or equal to a minimum operating voltage threshold *Vₜ* required by a controller (e.g. a master control unit, MCU) in the device 100, since as soon as the output voltage of the power source 101 falls below that level (i.e. *Vₜ*) the device 100 will no longer be operable (i.e. preventing the power source 101 from being further discharged) until the power source 101 has been charged to a higher level. The circuitry 102 may be configured to control the supply of electrical power to the at least one power source 101 according to a charging profile in which one or more of a charging voltage, a charging current, and a charging C-rate varies over time during the charging operation. Accordingly, the circuitry 102 may also be referred to as "charging circuitry" or "charging control circuitry". In some example embodiments, the charging current may be selected according to the cell's capacity and a charging C-rate that is known to be supported by the cell. For example, for a cell with a capacity of 200 milliamp hours (mAh) that is known to support a charging C-rate up to 25C, the circuitry 102 may be configured to control the supply of electrical power to the at least one power source 101 according to a charging profile in which the charging current is equal to or less than 5 Amperes, A (i.e. 0.2×25 = 5).

The charging profile may be configured specifically for the particular type of power source 101 used in the device 100. For example, in some example embodiments the at least one power source 101 may be configured to be removable and replaceable. In some such embodiments, the circuitry 102 may be configured to store a plurality of charging profiles, each associated with a different type of power source 101. Upon replacement of the at least one power source 101 with a replacement power source, the circuitry 102 may be configured to identify a type of the replacement power source (e.g. by communicating with the replacement power source to receive an identifier indicative of the power source type, or by communicating with a separate power source identification mechanism either local or remote to the non-combustible aerosol provision device 100). The circuitry 102 may then select one of the plurality of charging profiles associated with the identified type of the replacement power source during a subsequent charging operation, for more effective charging (e.g. faster and/or more reliable charging).

The non-combustible aerosol provision device 100 of the present embodiment is configured to receive a consumable 110 comprising aerosol-generating material 111 (e.g. a tobacco consumable 110, for example in the form of a tobacco stick). The at least one power source 101 is rechargeable, and can be recharged using electrical energy received via the charging interface 105. The at least one power source 101 provides power to the aerosol generator 103 to generate the aerosol, and may also provide power to other components of the non-combustible aerosol provision device 100 (e.g. active components within circuitry 102) during operation of the device 100. Depending on the embodiment, the charging interface 105 may comprise a wired interface or a wireless interface. In the case of wireless charging, the charging interface 105 may comprise a resonant receiver, for example in the form of an antenna. The antenna of the resonant receiver may be used to receive magnetic field energy for use in charging the at least one power source 101 (e.g. under the control of the circuitry 102).

The housing 105 can be configured to contain, hold, or otherwise support some or all of the other components of the non-combustible aerosol provision device 100. The housing 105 may be formed of any suitable material, including but not limited to paper, card, paperboard, cardboard, reconstituted material, a plastics material, a ceramic material, a composite material (e.g. carbon fibre), glass, a metal, or a metal alloy. In some embodiments the housing 105 can be configured so as to allow one or more components of the non-combustible aerosol provision device 100 to be removed and replaced. For example, the housing 105 may comprise a moveable or removable portion (e.g. in the form of a panel, cover, cap or plug) to enable access to components such as the circuitry 102, the at least one power source 101 and/or the aerosol generator 103, potentially to allow such components to be removed and replaced to repair or upgrade the device 100, or to allow such components to be removed prior to disposal of the device 100 (e.g. to allow different materials used in the device 100 to be separated for disposal/recycling). In some embodiments, the housing 105 is configured to enable the at least one power source 101 to be removed and replaced.

In the use of the device 100, the consumable 110 is inserted into a receiving portion of the housing 105, such that the consumable 110 may be heated by the aerosol generator 103 to generate an aerosol (and tobacco flavour, in the case of a tobacco consumable) for the user. When a user inhales at the end of the consumable 110, which may be referred to as a "puff", air is drawn into the device 100 through one or more air inlets and passes through the consumable 110, delivering the aerosol (and tobacco flavour, in the case of a tobacco consumable) to the user.

In the present embodiment, the aerosol generator 103 is an induction heater comprising an induction coil 103a and a susceptor 103b. In use, the circuitry 102 directs electrical energy from the power source 101 to the induction coil 103a, so as to cause a varying electrical current to flow through the induction coil 103a. This in turn creates a varying magnetic field which penetrates an electrically-conductive material of the susceptor 103b, causing magnetic hysteresis heating of the susceptor 103b. The consumable 110, and the aerosol-generating material 111 contained therein, is arranged to be heated by the susceptor 103b. In the present embodiment the susceptor 103b is provided in the device 100, for example within the housing 105. However, in other embodiments the susceptor 103b may be provided in the consumable 110.

Although in the present embodiment induction heating is used to generate an aerosol, in other embodiments a different mechanism may be used to generate the aerosol. For example, in some embodiments the non-combustible aerosol provision device 100 may still be configured to receive a consumable comprising tobacco (e.g. in the form of a tobacco stick) as shown in Fig. 1, but instead of an induction coil 103a and susceptor 103b the aerosol generator 103 may comprise a resistive heater. In embodiments in which tobacco is heated by a resistive heater, the aerosol generator 103 (i.e. the resistive heater) may be arranged so as to be in physical contact with at least some of the tobacco material contained in the consumable 110, when the consumable is received in the device 100. For example, this may be achieved by providing a resistive heater in the form of a protruding element, such as a spike or blade, that is pushed into the tobacco 111 in the process of inserting the consumable 110 into the device 100.

The non-combustible aerosol provision device 100 is described by way of example only. Many alternative aerosol provision devices may be used in example implementations of the principles described here. For example, instead of a tobacco heating system such as the device 100 illustrated in Fig. 1, in other embodiments a non-combustible aerosol provision device may be a vaping device in which an aerosol generating material (e.g. a liquid) is heated to generate the aerosol. The principles of the present disclosure are not limited to a particular type of aerosol provision device 100. That is to say, the aerosol provision device 100 may be arranged to aerosolise a solid, liquid or other aerosol-generating material via any suitable electrically powered or controller aerosol generator, such as a heater, a vibrating mesh, a source of irradiation, an electrically controller pressurised cannister which may include an electrically operated release valve, and so on.

Hence, in some embodiments the at least one aerosol generator 103 may be configured to generate an aerosol from the aerosol-generating material without heating, for example by using one or more of the following: vibration, pressure, or electrostatic energy. Examples of non-heating aerosol generators include an ultrasonic vaporiser, and a surface acoustic wave vaporiser. In embodiments in which the aerosol generator 103 is configured to generate the aerosol by heating the aerosol-generating material, the aerosol generator 103 may comprise any one of the following: an induction heater, an electromagnetic (EM) radiation heater (e.g. a laser heater, a non-laser optical heater, an infra-red heater, a radio-frequency heater, or a microwave frequency heater), a plasma heater, a microfluidic heater, a convection heater, a conduction heater, an electro-resistive heater (e.g. a graphene heater or ceramic heater), a halogen heater, a dielectric heater.

In embodiments in which the non-combustible aerosol provision device 100 is a so-called vaping device, the non-combustible aerosol provision device 100 may form part of an aerosol provision system comprising a modular assembly, often having two main functional parts, namely the aerosol provision device 100 and an article 110. In such embodiments the article 110 may comprise the consumable aerosol-generating material 111 and the aerosol generator 103 (e.g. a resistive or inductive heating element), while the aerosol provision device 100 part may comprise longer-life items, such as the rechargeable power source 101, circuitry 102, and other components such as user interface features. The aerosol provision device 100 may also be referred to as a reusable part or battery section, and the article 110 may also be referred to as a consumable, disposable/replaceable part, cartridge or cartomiser.

In both tobacco heating systems and vaping systems, the aerosol provision device 100 and article 110 may be mechanically coupled together at an interface for use, for example using a screw thread, bayonet, latched or friction fit fixing. When the aerosol-generating material in an article 110 has been exhausted, or the user wishes to switch to a different article having a different aerosol-generating material (e.g. a different composition, such as a different flavour), the article 110 may be removed from the aerosol provision device 100 and a replacement article may be attached to the device 100 in its place. Alternatively, in some embodiments an article 110 is configured such that, after the aerosol-generating material 111 in the article 110 has been exhausted, the article 110 can be refilled with more aerosol-generating material 111 (e.g. more tobacco in the case of a tobacco heating system, or more liquid in the case of a vaping systems), thereby allowing the article 110 to be reused. In some such embodiments, the user is able to refill the article 110 using a separate reservoir of aerosol-generating material 111. The aerosol-generating material used to refill the article may be the same or different to the previous aerosol-generating material in the article, thereby allowing the user to change to a different aerosol-generating material 111 without purchasing a new article 110.

As noted above, the at least one power source 101 is rechargeable. A system comprising the rechargeable non-combustible aerosol provision device 100 of Fig. 1 and an apparatus 200 for recharging the aerosol provision device will 100 now be described with reference to Fig. 2, according to an example embodiment. For example, the apparatus 200 for recharging the non-combustible aerosol provision device 100 may be referred to as a "charging apparatus", "charger", or a "charging case" or "charging pack", depending on its physical form. In Fig. 2 the apparatus 200 is shown in the form of a charging case or charging pack, comprising a housing 210 having a space in which the non-combustible aerosol provision device 100 may be received during charging. In such embodiments the apparatus 200 may perform a dual function, by acting as a case or pack for protecting the non-combustible aerosol provision device 100 (e.g. while it is being carried by a user) whilst simultaneously charging the power source 101 of the non-combustible aerosol provision device 100. In other embodiments the charging apparatus may have a different form, for example, as a mains adapter or power pack connectable to the charging interface 105 of the non-combustible aerosol provision device 100 via a suitable charging cable or via an induction charging interface.

The charging apparatus 200 of the present example embodiment comprises a charging power source 201, charging control circuitry 202, user interface 203, external power interface 204, and a device interface 205. The user interface 203 may, for example, be used to convey information to a user about a status of the charging apparatus 200 and/or the non-combustible aerosol provision device 100. In some embodiments the user interface 203 may be omitted. The charging control circuitry 202 is configured to control the flow of electrical power from the charging power source 201 and/or from the external power interface 204 to the non-combustible aerosol provision device 100 via the device interface 205, to recharge the power source 101 of the non-combustible aerosol provision device 100.

The charging power source 201 is configured to store energy that can be used to charge the power source 105 of the non-combustible aerosol provision device 100. For example, the charging power source 201 may comprise a battery, capacitor, supercapacitor or any other suitable power source capable of storing and providing electrical energy. In some embodiments the charging power source 201 may be omitted, for example when the charging apparatus 200 is embodied as a mains adapter configured to direct power from a mains electrical supply to the non-combustible aerosol provision device 100.

The external power interface 204 is configured to receive power from an external power source, for example the mains electrical supply or another electrical device such as a tablet, laptop or desktop computer, smartphone, and so on. In some embodiments the external power interface 204 may be omitted, for example when the charging apparatus 200 is embodied as a charging case or charging pack. In such embodiments, when the external power interface 204 is omitted, the charging power source 201 may for instance be removable to allow the charging power source 201 to be replenished once depleted, either by replacing the charging power source 201 with another charging power source 201 that is partly or fully charged, or by recharging the charging power source 201 outside of the charging apparatus 200.

Although in the example embodiment shown in Fig. 2, the non-combustible aerosol provision device 100 is recharged using the charging apparatus 200, in other embodiments a non-combustible aerosol provision device 100 may be recharged from any suitable source of electrical energy. For example, the charging interface 105 of the non-combustible aerosol provision device 100 may be a standardised interface such as a Universal Serial Bus-C (USB-C) type connector via which the non-combustible aerosol provision device 100 can be connected to and recharged from any device capable of providing power via a USB-C interface. It should be noted that a USB-C interface is described here purely by way of an illustrative example, and should not be construed as limiting. In other example embodiments, the non-combustible aerosol provision device 100 may comprise a different type of charging interface other than USB-C.

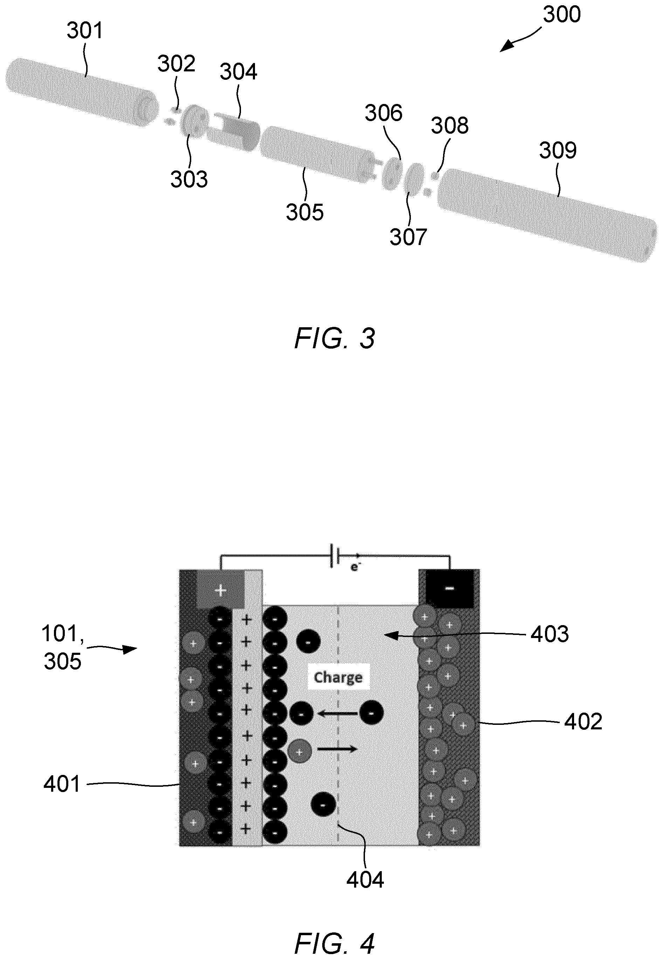

Turning now to Fig. 3, a non-combustible aerosol provision device 300 is illustrated according to another example embodiment. Whereas the non-combustible aerosol provision device 100 described above with reference to Figs. 1 and 2 is shown in the form of a tobacco heating system, configured to receive a consumable comprising a tobacco stick, in the example embodiment of Fig. 3 the non-combustible aerosol provision device 300 is configured to generate aerosol from aerosol-generating material in the form of a liquid substrate. Hence, the non-combustible aerosol provision device 300 shown in Fig. 3 may be referred to as a 'vaping device' or 'e-cigarette'.

The non-combustible aerosol provision device 300 comprises electrical contacts 302 (e.g. in the form of contact springs, contact pads or pogo pins) and an article interface 303. The article interface 303 is configured to engage with an article 301 so as to physically connect the article 301 to the non-combustible aerosol provision device 300. Although not shown in Fig. 3, the article 301 comprises a reservoir and an aerosol generator configured to generate an aerosol from liquid held in the reservoir. When the article interface 303 is engaged with the article 301, the electrical contacts 302 of the non-combustible aerosol provision device 300 form an electrical connection to corresponding contacts on the article 301, thereby to supply power from the non-combustible aerosol provision device 300 to the aerosol generator in the article 301 to generate an aerosol. In some embodiments the non-combustible aerosol provision device 300 may be configured to supply electrical power wirelessly to the aerosol generator, in which case the electrical contacts 302 may be omitted. For example, in such embodiments the non-combustible aerosol provision device 300 may comprise an induction coil, and the aerosol generator 301 in the article may comprise a susceptor configured to generate heat when subjected to a varying magnetic field generated by the induction coil, as described above. As a further alternative, in some embodiments the article 300 may not comprise an aerosol generator. For example, both an induction coil and a susceptor may be included in the non-combustible aerosol provision device 300, removing the need for a separate aerosol generator in the article 300.

Continuing with reference to Fig. 3, the non-combustible aerosol provision device 300 comprises article interface circuitry 304, which in the present embodiment is in the form of a flexible printed circuit board (flexi-PCB), a power source 305, charging circuitry 306, connector 307, charging contacts 308 and housing 309. The article interface circuitry 304 and the charging circuitry 306 may perform similar functions to the circuitry 102 of the device 100 described above with reference to Figs. 1 and 2. The power source 305 may perform similar functions to the power source 101 of the device 100 of Figs. 1 and 2, such as providing power to the aerosol generator to generate the aerosol, and providing power to other components of the non-combustible aerosol provision device 300 during operation of the device 300. The connector 307 and charging contacts 308 may perform similar functions to the charging interface 105 of the device 100 of Figs. 1 and 2.

Once assembled, the non-combustible aerosol provision device 300 is elongate and substantially tubular in shape. Depending on the dimensions and proportions of the housing 309, in some example embodiments the non-combustible aerosol provision device 300 may have an appearance similar to that of a conventional cigarette, for example a king-size (KS) factory-made cigarette (FMC).

As described above with reference to Figs. 1 to 3, a non-combustible aerosol provision device 100, 300 for providing an aerosol during a puff comprises a power source 101, 305 configured to supply electrical power to at least one aerosol generator. In some example embodiments the power source 101, 305 is a supercapacitor, for example a hybrid supercapacitor such as a lithium-ion capacitor (LIC).

In the present disclosure, the term "hybrid supercapacitor" is used to refer to a cell in which one electrode (e.g. an electrode comprising activated carbon) primarily stores energy through an adsorption process via the electric double layer formed on a surface of the electrode, whilst the other electrode (e.g. an electrode comprising graphite pre-doped with lithium) primarily stores energy via intercalation of lithium ions. Conversely, it will be understood that the electrode that primarily stores energy through adsorption via the electric double layer will release energy via a desorption process, whilst the electrode that primarily stores energy via intercalation of lithium ions will release energy via a deintercalation process. The electrode that primarily stores charge as an electric double layer is referred to herein as the cathode, whilst the electrode that primarily stores charge via intercalation of lithium ions is referred to herein as the anode.

Here, the term "primarily stores" means that the mechanism in question is the dominant mechanism by which charge is stored on or in that electrode during operation of the cell, but does not exclude the possibility that a certain amount of charge may also be stored by one or more other mechanisms. For instance, in some example embodiments the cathode (i.e. the electrode that primarily stores energy through adsorption via the electric double layer) may also store and release energy via intercalation/deintercalation of lithium ions. For example, in such embodiments the cathode may comprise a transition metal oxide (TMO) material capable of absorbing and releasing lithium ions via intercalation/deintercalation. In some example embodiments, the cathode may comprise a combination of activated carbon and TMO material.

Depending on the embodiment, various different materials and combinations of materials may be used in the anode and the cathode of the hybrid supercapacitor power source 101. The cathode may, for example, comprise or consist of one or more of the following materials: activated carbon, heteroatom-doped carbon (e.g. nitrogen, phosphorus doped) or graphene, with dopants including but not limited to lithium cobalt oxide (LCO), lithium manganese oxide (LMO), lithium iron phosphate (LFP), lithium iron manganese phosphate (LMFP), lithium nickel aluminium oxide (NCA), and lithium nickel manganese cobalt oxide (NMC). The anode may, for example, comprise or consist of one or more of the following materials: lithium pre-doped graphitic carbon, hard carbon and or soft carbon, lithium titanium oxide (LTO), or iron oxide (Fe₂O₃). Here, the terms "hard carbon" and "soft carbon" both refer to amorphous forms of carbon, in other words, forms of carbon that have a low degree of graphitization within the structure. The main difference between hard and soft carbon is that soft carbon can be fully converted to graphite when heat treated up to 3000 °C, whereas hard carbon cannot be converted to graphite when heat treated up to 3000 °C.

In some example embodiments the cathode and/or the anode comprises one or more cation dopants, including but not limited to: sodium (Na), potassium (K), magnesium (Mg), zinc (Zn), calcium (Ca), iron (Fe), aluminum (Al), yttrium (Y), chromium (Cr), molybdenum (Mo), tellurium (Te), zirconium (Zr), silicon (Si), tin (Sn), titanium (Ti), ruthenium (Ru), vanadium (V), tungsten (W), tantalum (Ta), gallium (Ga), and niobium (Nb). For example, dopants such as Ca, Y, Cr, Mo and Ti may be added to the cathode and/or the anode to improve the cycle life of the supercapacitor. In some example embodiments the cathode and/or the anode comprises one or more anion dopants, including but not limited to: fluorine (F), chlorine (Cl), sulfur (S), nitrogen (N), silicon (Si), boron (B), and phosphorus (P).

The addition of one or more dopant species to the anode and/or the cathode, such as the dopants listed above, can offer a number of advantages compared to non-doped LIC hybrid supercapacitors. For example, the addition of one or more dopants to the anode and/or the cathode can eliminate adverse surface defects, thereby improving the structural stability and electrochemical performance of the anode or cathode materials.

A hybrid supercapacitor, such as an LIC, also contains a liquid electrolyte and a separator film. The separator film is disposed between the anode and the cathode so as to separate the anode and the cathode. In other words, the separator film prevents the anode from coming into contact with the cathode, which would result in a short-circuit. The separator film permits the flow of electric charges through the electrolyte and through the separator film, such that the electric charges that are transported in the electrolyte may flow from the anode to the cathode and vice versa.

In example embodiments, the liquid electrolyte may contain one or more of the following: a lithium salt, including but not limited to lithium hexafluorophosphate (LiPF₆), lithium hexafluoroarsenate (LiAsF₆), lithium bis (fluorosulfonyl) imide (LiFSI), lithium bis (trifluoromethanesulfonyl) imide (LiTFSI), lithium tetrafluoroborate (LiBF₄), lithium perchlorate (LiClO₄), and lithium bis(oxalato) borate (LiBOB); a carbonate-based organic solvent, including but not limited to ethylene carbonate (EC), propylene carbonate (PC), ethyl methyl carbonate (EMC), diethyl carbonate (DEC), dimethyl carbonate (DMC), propyl acetate (PA), methyl formate (MF), methyl acetate (MA), ethyl acetate (EA), methyl pyrrolidone (MP), tetrahydrofuran (THF), dimethylsulfoxide (DMSO), 1,2-dimenthoxyethane( DME), and dichloromethane (DCM); a nitrile-based organic solvent, such as acetonitrile and/or acrylonitrile; and one or more combinations of additives, including but not limited to vinylene carbonate (VC), vinyl ethylene carbonate (VEC), and fluoroethylene carbonate (FEC).

In example embodiments, the separator film comprises one or more of the following: cellulose or a cellulose-derived material; a monolayer or multilayer polyolefin-based microporous film, including but not limited to polypropylene, polyethylene, polyvinyl chloride and/or poly(tetrafluoroethylene); nonwoven fibres; naturally occurring substances; and ceramic.

In example embodiments, a supercapacitor that is used as a power source in a non-combustible aerosol provision device (e.g. the power source 101 in the device 100 of Fig. 1, or the power source 305 in the device 300 of Fig. 3) may be in the form of a cell having any one of the following cell shapes: a cylindrical cell shape, a pouch cell shape, a rectangular cell shape, and a prismatic cell shape.

Referring now to Fig. 4, an electrode structure of a hybrid supercapacitor power source is schematically illustrated, according to an example embodiment. In the example embodiment of Fig. 4, the hybrid supercapacitor is a LIC. The power sources 101, 305 of the non-combustible aerosol provision devices 100, 300 described above with reference to Figs. 1 to 3 may comprise a hybrid supercapacitor such as the one illustrated in Fig. 4. The LIC hybrid supercapacitor power sources 101, 305 may be configured to be removeable and/or replaceable from the aerosol provision device, as has been described above with reference to Fig. 1.

The LIC power source 101, 305 of Fig. 4 comprises a cathode 401, an anode 402, a liquid electrolyte 403, and a separator film 404. The arrows in Fig. 4 illustrate the direction of movement of electric charges (e.g. cations, anions, electrons etc.) during a process of charging the LIC. In the present example embodiment, the cathode 401 comprises activated carbon doped with a TMO, for example NMC, LCO, LFP and/or LTO, whilst the anode 402 comprises lithium pre-doped graphite. During charging, electric charge is stored at the cathode 401 in the form of an electric double layer on the surface of the cathode 401 and in the form of intercalated ions, whilst electric charge is stored at the anode 402 in form of intercalated lithium ions.

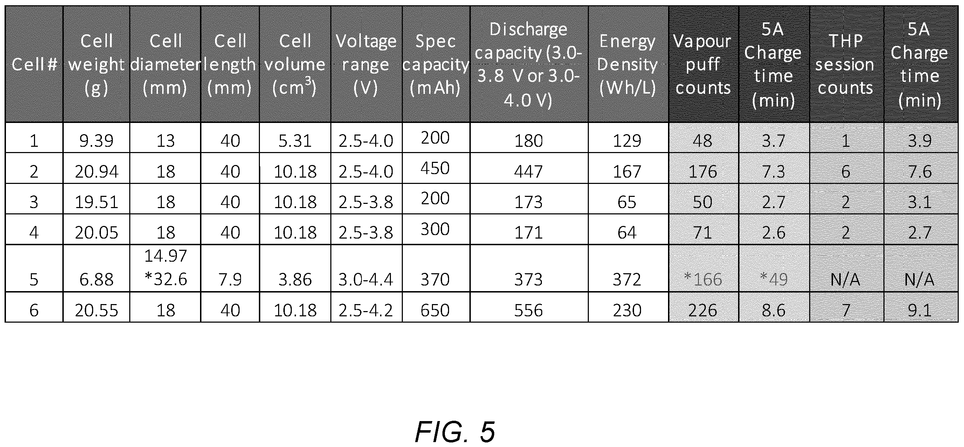

Figure 5 is a table illustrating cell properties and test results for six different types of power sources (cells), including LIC hybrid supercapacitor cells according to example embodiments. The table lists the following properties for each cell:
- Cell weight in grams (g);
- Cell diameter in millimetres (mm) - note that a single diameter is stated for cells 1-4 and 6 since these are circular in cross-section, whilst two values are stated for cell 5 since this has a rectangular cross-section;
- Cell length in mm;
- Cell volume in cubic centimetres, cm³;
- Operating voltage range of the cell, in volts (V);
- The manufacturer's specified capacity of the cell, in milliampere-hours (mAh); and
- The energy density of the cell, measured in Watt hours per litre (Wh/L).

In addition, the table in Fig. 5 lists the following test results for each cell:
- The discharge capacity of the cell, which is the total energy discharged from the cell (measured in mAh) during a discharge operation in which the cell voltage drops from a first voltage to a second voltage, the second voltage being lower than the first voltage;
- The vapour puff counts, which is the total number of puffs that can be achieved based on the discharge capacity and a simulated power consumption profile for an example vaping device;
- The 5 Ampere (A) charge time for the example vaping device comprising the cell, measured in minutes (min), which is the time taken to charge the cell from a discharged state to a charged state (in this case, charging from a discharged state in which the output voltage of the cell is too low for a controller in the device to operate up to a fully charged state, i.e. 100% state of charge, SOC);
- The THP session counts, which is the total number of sessions that can be achieved based on the discharge capacity and a simulated power consumption profile for an example THP (tobacco heated product) device; and
- The 5A charge time for the example THP device comprising the cell, measured in minutes (min), which is the time taken to charge the cell from a discharged state to a charged state (in this case, charging from a discharged state in which the output voltage of the cell is too low for a controller in the device to operate up to a fully charged state, i.e. 100% state of charge, SOC).

In each of the tests set out above, for which the results are given in Fig. 5, the cell in question was either charged or discharged between a certain discharged state and a certain charged state. Here, the term "discharged state" refers to the state of charge of the cell at the start of the test (for tests involving charging) or at the end of the test (for tests involving discharging). Conversely, the term "charged state" refers to the state of charge of the cell at the end of the test (for tests involving charging) or at the start of the test (for tests involving discharging). The charged state in all of the tests for which results are given in Fig. 5 was a fully charged state, meaning a state of charge in which the cell is considered to no longer capable of storing any further electrical charge (which may also be referred to as a 100% SOC). For example, the cell may be considered to be in the fully charged state once the level of current flowing to the cell (in the case of charging) falls below a minimum threshold, e.g. 0.5A. The discharged state in all of the tests for which results are given in Fig. 5 was a state of charge in which the output voltage of the cell is below (e.g. marginally below) a minimum operating voltage threshold *Vₜ* required by a controller (e.g. a master control unit, MCU) in the device. In other words, the discharged state in all tests was defined as being a state of charge in which output voltage of the cell had fallen to a level at which the controller in the device was no longer able to operate, meaning that the device was rendered inoperable with the cell in the discharged state.

In the results shown in Fig. 5, the measured charge time at a charging current of 5A for each cell is longer when used in the example THP device, compared to the measured charge time at the same charging current (5A) for the same cell in the example vaping device. This is because the example THP device and the example vaping device used in these tests comprised different controllers having different minimum operating voltages. The controller in the example THP device has a minimum operating voltage of 3.0 V, whereas the controller in the example vaping device has a minimum operating voltage of 3.3 V. Hence, the starting point in the 5A charge time tests for the example THP device was a discharged state in which the output voltage of the cell was at (or marginally below) 3.0 V, whereas the starting point in the 5A charge time tests for the example THP device was a discharged state in which the output voltage of the cell was at (or marginally below) 3.3 V. In both cases, the end point of the 5A charge time tests was when the cell reaches its maximum output voltage (i.e. at a 100% state of charge). For this reason, the measured 5A charge times are longer for the example THP device compared to the measured 5A charge times for the example vaping device.

Cells #1 to #4 in the table shown in Fig. 5 comprise LIC power sources according to example embodiments. Specifically, cells #1, #2, #3 and #4 are LIC cells comprising an activated carbon cathode and a lithium pre-doped graphite anode. Cells #3 and #4 comprise cathodes having a lower level of TMO dopants compared to cells #1 and #2. Cell #5 is a comparative example of a commercially available vaping device comprising a conventional lithium-ion battery, and cell #6 is a comparative example of a commercially available vaping device comprising an LIC power source.

Cell #1 is an LIC cell manufactured by CDA (RTM), identified by the product code LIB1340Q4R0507. Cell #2 is an LIC cell manufactured by CDA, identified by the product code LIB1840Q4R0118. Cell #3 is an LIC cell manufactured by CDA, identified by the product code LIC1840Q3R8507. Cell #4 is an LIC cell manufactured by CDA, identified by the product code LIC1840Q3R8757. Based on the test results presented herein, the present inventors have recognised that such cells are particularly suited for use as the power source in an aerosol provision system, as will be explained below.

The values given for the discharge capacities in Fig. 5 were obtained at a C-rate of 0.2C and at a temperature of 25 °C. C-rates are widely used to allow comparisons between cells of different capacities, and a C-rate of 0.2C means that the cell would be fully discharged after 5 hours (1/0.2) if power was drawn from the cell at a continuous rate of 0.2C, starting with the cell fully-charged. For cells #1 and #2, both of which have a specified operating voltage range of 2.5-4.0 V, as stated in the column headed "Voltage range" in Fig. 5, the discharge capacity was measured over the voltage range 3.0-4.0 V. For cells #3 and #4, both of which have a specified operating voltage range of 2.5-3.8 V, as stated in the column headed "Voltage range" in Fig. 5, the discharge capacity was measured over the voltage range 3.0-3.8 V so as not to exceed the maximum operating voltage of the cell.

The values given for the vapour puff counts and THP session counts in Fig. 5 were obtained based on simulated power consumption profiles for an example vaping device. The power consumption profile was determined by repeatedly subjecting the device to puffs of 3-second duration with a 30-second pause between puffs, under the control of a smoke engine to ensure repeatability. During each puff, power was supplied to the aerosol generator by the power source using pulse width modulation (PWM), with a 50% duty cycle and a switching frequency of 50 Hertz (Hz). Under these conditions, the example vaping device consumes approximately 6.5 milliWatt hours (mWh), or 23.4 Joules (J), per puff. The power consumption profile was then translated to a battery test script and applied to each of the cells under test (cells #1 to #6) using a Maccor battery tester, starting with the cell in the fully-charged state. The number of simulated puffs was then counted until the cell was fully depleted, to determine the total number of puffs that can be supplied by the cell when fully-charged (i.e. the "vapour puff counts" value).

A similar approach was used to obtain the values for the THP session counts in Fig. 5, which were obtained based on simulated power consumption profiles for an example tobacco heating system. In this case, the aerosol generator in the tobacco heating system is activated for a longer period of time (referred to as a "session") in comparison to the aerosol generator in the vaping device, which is activated at the start of each puff and deactivated at the end of each puff. A user will typically take several puffs on a tobacco heating system during the session. In the present example, the power consumption profile for the example tobacco heating system was obtained based on an average session duration of approximately 4 minutes and 45 seconds (4.75 min), during which the tobacco heating system consumes approximately 220 mWh, or 792 J, of power from the cell. The power consumption profile was then translated to a battery test script and applied to each of the cells under test (cells #1 to #6) using a Maccor battery tester, starting with the cell in the fully-charged state. The number of simulated THP sessions was then counted until the cell was fully depleted, to determine the total number of sessions that can be supplied by the cell when fully-charged (i.e. the "THP session counts" value). When determining the THP session counts, if the cell became depleted part-way through a simulated session (i.e. giving a non-integer value of the THP session count), then the number of sessions is rounded down to the nearest integer so as to give the total number of complete sessions that can be provided by a fully-charged cell.

Historically, the trend in aerosol provision systems (e.g. vaping devices and tobacco heating systems) has been to increase the capacity of the device's power source to allow the device to be used for a longer period of time in between charges. At the same time, there has also been a desire to shorten the charging time for the device's power source, so that the device can be recharged more quickly once the power source has been depleted. These represent conflicting technical requirements, since as the capacity is increased the charging time will increase commensurately. The example of cell #6 in Fig. 5, which is from a commercially available LIC-powered vaping device, follows this trend by using a relatively high-capacity LIC power source (650 mAh capacity).

However, the present inventors have recognised that since supercapacitors can be charged much more rapidly than conventional batteries (e.g. lithium-ion batteries), when using a supercapacitor as a power source in an aerosol provision system the total capacity becomes less significant from the user's perspective. This is because when the cell is depleted, the much faster charge time means that the device can be quickly recharged and be ready to use once more, with minimal inconvenience for the user. In other words, the act of recharging the device is much less of an inconvenience for a user when a supercapacitor power source is used, compared to devices comprising conventional power sources.

The present inventors have recognised that using a supercapacitor as the power source in an aerosol provision device gives an opportunity to reduce the capacity (and hence the vapour puff count or THP session count) without causing any significant inconvenience for the user, since the charge time for a lower-capacity supercapacitor (e.g. cells #1, #3 and #4 in Fig. 5) can be much lower than the charge time for a conventional battery (e.g. cell #5), or even for a larger-capacity supercapacitor (e.g. cell #6). In other words, rather than following the historical trend of increasing the cell's capacity compared to previous generations of devices, using a supercapacitor as the power source in an aerosol provision system offers an opportunity to reduce the charging time yet further (i.e. the time taken to fully charge the cell, from 0% to 100%), by prioritising a reduction in charging time versus prioritising the number of puffs/sessions between charges.

Hence, in some example embodiments, an aerosol provision system may comprise a power source comprising at least one supercapacitor (e.g. a hybrid supercapacitor, such as an LIC) having a charging time of less than or equal to 12 minutes. Here, the term "charging time" should be understood as meaning the time taken to fully charge the cell, from 0% to 100%. In such embodiments, the reduction in charging time compared to prior art devices is achieved in part by using a supercapacitor as opposed to e.g. a conventional lithium-ion battery, and in part by selecting a supercapacitor cell of lower capacity to reduce the charging time still further. Hence, in such embodiments, as well as having a charging time of less than or equal to 12 minutes the supercapacitor may also have a capacity such that in the fully-charged state (i.e. when 100% charged) the supercapacitor is configured to store sufficient electrical charge to supply electrical power to the at least one aerosol generator for no more than 80 puffs.

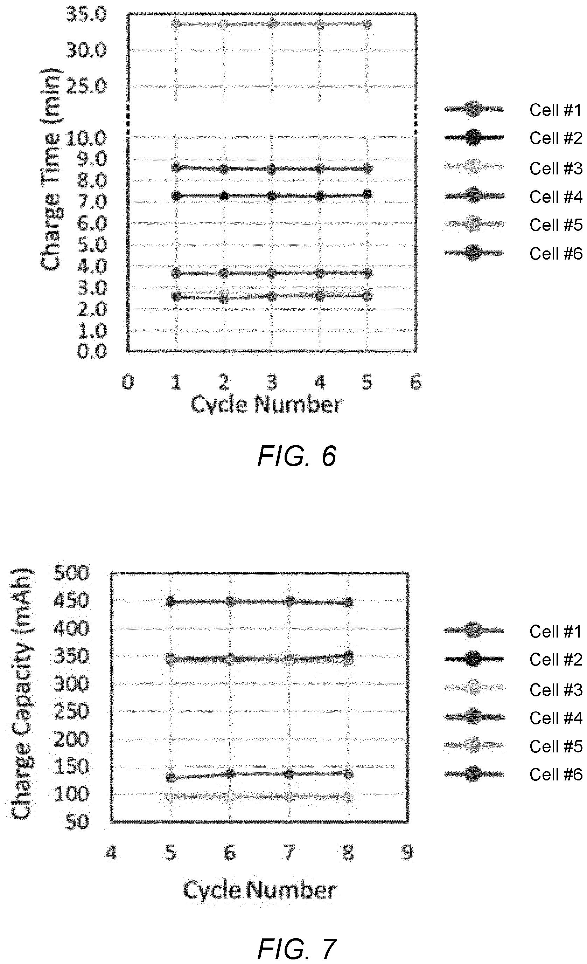

This advantage is demonstrated in Fig. 6, which plots the 5A charge time in minutes against the cycle number (number of charge/discharge cycles) for cells #1 to #6. The charge time can be understood as the time taken for a cell to be charged from one state to another state. As described above with reference to Fig. 5, the starting point of the cell when measuring the charge time may be referred to as a "discharged state", and the end point (i.e. after charging has been completed) may be referred to as a "charged state". It will be appreciated that the discharged state and charged state may be defined differently depending on the test criteria used to measure a charging time. In the present example embodiments, the charge time was measured when starting with the cell in a state of charge (SOC) in which its output voltage was at (or marginally below) the minimum voltage required by a controller in the device to operate. This starting point may be referred to as the "discharged state" or even the "fully discharged state", although in the latter case it should be appreciated that in reality the cell will still hold a finite amount of charge even when the output voltage drops below the minimum operating voltage required by the device's controller. Similarly, the charged state may be understood as being one in which the cell is unable to store any more electrical charge, and may therefore be referred to as a "fully charged state" or 100% SOC.

In the present example embodiments, the 5A charge time for cells #1 to #4 and #6 was measured by charging the cell at a constant current of 5A until the output voltage of the cell reached the corresponding upper limit specified in the "Voltage Range" column in Fig. 5, and then continuing charging at constant voltage (i.e. whilst holding the voltage at the upper limit) until the current reduced to 0.5A. For cell #5, due to limitations with the example vaping device that was used, the charge time was measured by charging the cell at a constant current of 1.11A (equal to a C rate of 3C for cell #5) until the output voltage of the cell reached 4.4V, and then continuing charging at a constant voltage of 4.4V until the current reduced to 0.11A (0.1C rate).

As shown in Fig. 6, cells #1, #3 and #4 all have a significantly shorter charge time than cells #5 and #6, and therefore offer greater convenience for a user by reducing the length of time that the user must wait before the device is fully charged. In particular, each of cells #1, #3 and #4 has a vapour puff count of 80 puffs or less, meaning that when fully charged the cell is configured to store sufficient electrical charge to supply electrical power to the at least one aerosol generator for no more than 80 puffs. Additionally, cells #1, #3 and #4 each have a 5A charge time of less than or equal to 12 minutes. In some example embodiments, the supercapacitor may have a charge time of less than or equal to 10, 9, 8, 7, 6, 5 or 4 minutes (e.g. cells #1, #3 and #4 all have a 5A charge time of 3.7 minutes or less). In some example embodiments, the supercapacitor may have a vapour puff count of 50 puffs or less (e.g. cells #1 and #3 have vapour puff counts of 48 and 50 puffs respectively).

By utilising a lower-capacity supercapacitor in this way, to further reduce the charge time, in some example embodiments an aerosol provision device may therefore comprise a power source comprising at least one supercapacitor (e.g. a hybrid supercapacitor such as an LIC) having a total energy storage capacity of less than or equal to 450 mAh, as is the case for each of cells #1 to #4 in Fig. 5. In some such embodiments, the total energy storage capacity may be between 200 and 300 mAh, as is the case for cells #1, #3 and #4.

Similarly, by utilising a lower-capacity supercapacitor in this way, the volume and/or mass of the cell may be further reduced, enabling a more compact and/or lightweight device. For instance, in some example embodiments an aerosol provision device may comprise a power source comprising at least one supercapacitor (e.g. a hybrid supercapacitor such as an LIC) having a mass less than or equal to 20 grams (e.g. cell #1 has a mass of 9.39 g, whilst cell #3 has a mass of 19.51 g). In some example embodiments an aerosol provision device may comprise a power source comprising at least one supercapacitor (e.g. a hybrid supercapacitor such as an LIC) having a volume less than or equal to 10 cm³, for instance less than or equal to 9, 8, 7, or 6 cm³ (e.g. cell #1 has a volume of 5.31 cm³).

As described above, the charge time of an aerosol provision system can be reduced by using a supercapacitor of relatively low capacity as the power source for the aerosol generator. In some example embodiments, a supercapacitor cell can be selected based on its discharge capacity or charge capacity, and based on the vapour puff count that can be achieved by that cell in the intended aerosol provision system. For example, in some embodiments an aerosol provision system comprises at least one power source comprising a supercapacitor, wherein the supercapacitor has a discharge capacity between a first output voltage limit and a second output voltage limit of no more than 180 mAh, and in the charged state the supercapacitor is configured to store sufficient electrical charge to supply electrical power to the at least one aerosol generator for no more than 80 puffs.

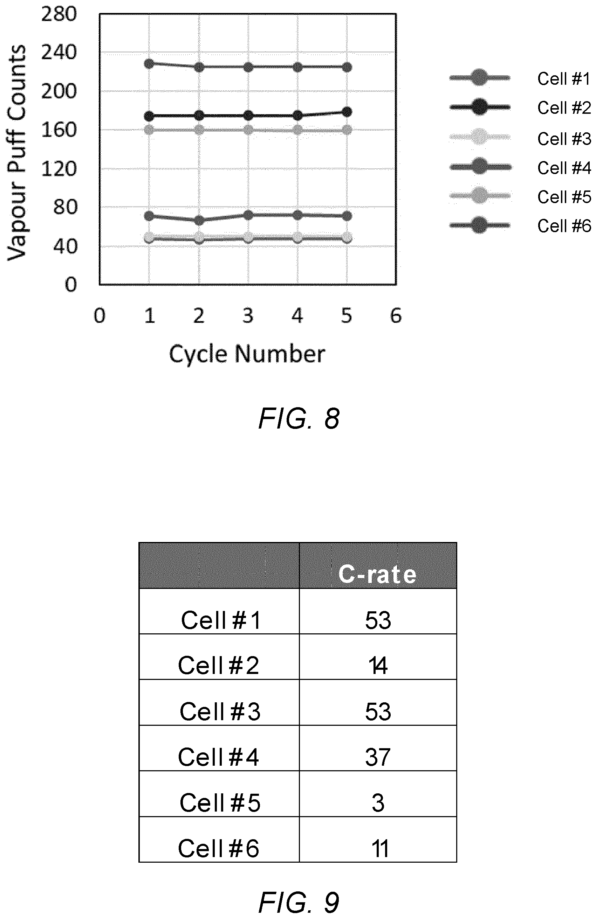

For example, as shown in Fig. 5 cells #1, #3 and #4 all have discharge capacities of less than 180 mAh when measured at a C-rate of 0.2C (equivalent to a current <1A for each of cells #1, #3 and #4). Also, as shown in Fig. 7 cells #1, #3 and #4 all have charge capacities of less than 150 mAh (as measured at a charging current of 5A). At the same time, as shown in Fig. 8, cells #1, #3 and #4 each provide a vapour puff count of no more than 80 puffs when tested under the conditions specified above in relation to Fig. 5 (i.e. puffs of 3-second duration with a 30-second pause between puffs).

In some example embodiments the cell may provide a higher vapour puff count than 80 puffs when tested under these conditions, for example no more than 90, 100, 110, 120, 130, 140, 150, 160, 170, 180, 190 or 200 puffs (e.g. cell #2 provides a vapour puff count of 176 puffs). Furthermore, in some example embodiments the cell may provide a lower vapour puff count than 80 puffs, for example no more than 50, 60, or 70 puffs (e.g. both cells #1 and #3 provide vapour puff counts of no more than 50 puffs).

Figure 9 illustrates a table which lists charging rates (C-rates) that have been demonstrated for each of the six cells listed in Fig. 5, i.e. cells #1 to #6. As shown in Fig. 9, cells #1, #3 and #4 in particular can support much higher C-rates compared to other cells, notably cells #5 and #6, partly by virtue of their lower capacities compared to cells #5 and #6 (i.e. since the equivalent C-rate for a given current depends on the capacity of the cell). A higher C-rate equates to a lower charge time, hence, cells #1, #3 and #4 can be charged to their capacities more quickly compared to cells #5 and #6 since they can be charged at higher C-rates.

By selecting a supercapacitor cell based on one or more of the criteria described above with reference to Figs. 5 to 9, for use as the power source in an aerosol provision system, the charge time of the aerosol provision system can be further reduced.

Example embodiments have been described in which an aerosol provision system comprises at least one power source comprising at a supercapacitor, for example as described above with reference to Figs. 1 to 3 and cells #1 to #4 in Figs. 5 to 9. Supercapacitors are highly durable compared to alternative technologies, and can operate for in excess of 10,000 cycles. In some such embodiments, the at least one supercapacitor is configured to be operable for a number of charge and discharge cycles greater than or equal to 10,000 cycles, and in some embodiments greater than or equal to 20,000 cycles. The maximum number of charge and discharge cycles to which a cell can be subjected before the cell's performance degrades beyond a defined limit (e.g. a point at which the cell's capacity falls below a certain percentage of its starting capacity when new, or a point at which the cell fails) may be referred to as the "cycle life" or "operating life" of the cell. In some example embodiments, after said number of charge and discharge cycles (e.g. 10,000 cycles, or 20,000 cycles) the at least one supercapacitor is still capable of storing sufficient electrical charge to supply electrical power to the at least one aerosol generator for at least 80 puffs.

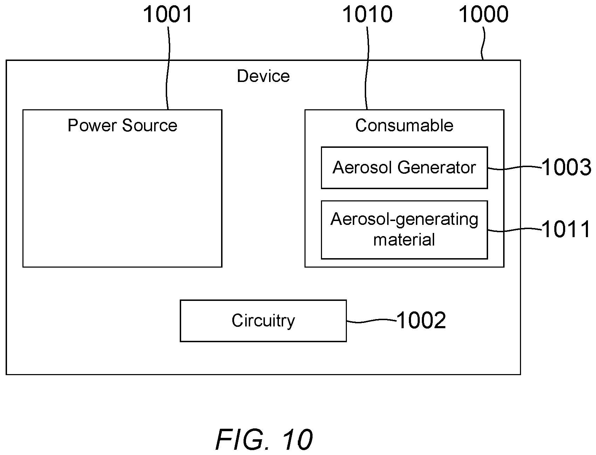

Example embodiments have therefore been described in which an aerosol provision device comprises least one power source comprising at least one supercapacitor configured to supply electrical power to at least one aerosol generator for generating the aerosol from aerosol-generating material. An example of such an aerosol provision device is schematically illustrated in Fig. 10. The device 1000 comprises at least one supercapacitor power source 1001, and circuitry 1002 (e.g. similar to the circuitry 102 described above with reference to Fig. 1 or the circuitry 304, 306 described above with reference to Fig. 3). The circuitry 1002 can control the flow of power from the supercapacitor power source to an aerosol generator 1003, for generating an aerosol from an aerosol-generating material 1011. Depending on the embodiment, the aerosol generator 1003 and/or the aerosol-generating material 1011 may be included in the aerosol provision device 1000, or may be included in a physically separate consumable 1010 that is removeably engageable with the device 1000, so that the consumable can be removed and replaced as and when necessary (e.g. when the aerosol-generating material 1011 has been used up, or if the aerosol generator 1003 is no longer operable).

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc, other than those specifically described herein. In addition, this disclosure may include other inventions not presently claimed, but which may be claimed in future.

Aspects of the present invention are set out below in the following numbered clauses, which form part of the description:
Clause 1. An aerosol provision device for providing an aerosol during a puff on the aerosol provision device, the aerosol provision device comprising:
   at least one power source comprising at least one supercapacitor configured to supply electrical power to at least one aerosol generator for generating the aerosol from aerosol-generating material,
   wherein the at least one supercapacitor is a lithium-ion capacitor, LIC, comprising an anode and a cathode, and wherein said anode and/or said cathode comprises one or more dopants.
Clause 2. The aerosol provision device of clause 1, wherein said cathode comprises one or more of: activated carbon, heteroatom-doped carbon, or graphene.
Clause 3. The aerosol provision device of clause 1 or 2, wherein said cathode comprises one or more of the following as said one or more dopants: lithium cobalt oxide, LCO; lithium manganese oxide, LMO; lithium iron phosphate, LFP; lithium iron manganese phosphate, LMFP; lithium nickel aluminium oxide, NCA; and lithium nickel manganese cobalt oxide, NMC.
Clause 4. The aerosol provision device of clause 1, 2 or 3, wherein said anode comprises one or more of: lithium pre-doped graphitic carbon; hard carbon and/or soft carbon; lithium titanium oxide, LTO; and iron oxide, Fe₂O₃.
Clause 5. The aerosol provision device of any one of clauses 1 to 4, wherein said one or more dopants include one or more cation dopants.
Clause 6. The aerosol provision device of clause 5, wherein said one or more cation dopants comprise one or more of: Na; K; Mg; Zn; Ca; Fe; Al; Y; Cr; Mo; Te; Zr; Si; Sn; Ti; Ru; V; W; Ta; Ga; and Nb.
Clause 7. The aerosol provision device of any one of clauses 1 to 6, wherein said one or more dopants include one or more anion dopants.
Clause 8. The aerosol provision device of clause 7, wherein said one or more anion dopants comprise one or more of: F; Cl; S; N; Si; B; and P.
Clause 9. The aerosol provision device of any one of clauses 1 to 8, wherein the at least one supercapacitor comprises a liquid electrolyte.
Clause 10. The aerosol provision device of clause 9, wherein the liquid electrolyte comprises one or more of the following: a lithium salt; lithium hexafluoroarsenate, LiAsF₆; lithium bis (fluorosulfonyl) imide, LiFSI; lithium bis (trifluoromethanesulfonyl) imide, LiTFSI; lithium tetrafluoroborate, LiBF₄; lithium perchlorate, LiClO₄; and lithium bis(oxalato) borate, LiBOB.
Clause 11. The aerosol provision device of clause 10, wherein the lithium salt comprises lithium hexafluorophosphate, LiPF₆.
Clause 12. The aerosol provision device of any one of clauses 9 to 11, wherein the liquid electrolyte comprises a carbonate-based organic solvent.
Clause 13. The aerosol provision device of clause 12, wherein the carbonate-based organic solvent comprises one or more of: ethylene carbonate, EC; propylene carbonate, PC; ethyl methyl carbonate, EMC; diethyl carbonate, DEC; dimethyl carbonate, DMC; propyl acetate, PA; methyl formate, MF; methyl acetate, MA; ethyl acetate, EA; methyl pyrrolidone, MP; tetrahydrofuran, THF; dimethylsulfoxide, DMSO; 1,2-dimenthoxyethane, DME; and dichloromethane, DCM.
Clause 14. The aerosol provision device of any one of clauses 9 to 13, wherein the liquid electrolyte comprises a nitrile-based organic solvent.
Clause 15. The aerosol provision device of clause 14, wherein the nitrile-based organic solvent comprises acetonitrile and/or acrylonitrile.
Clause 16. The aerosol provision device of any one of clauses 9 to 11, wherein the liquid electrolyte comprises one or more additives.
Clause 17. The aerosol provision device of clause 16, wherein said one or more additives comprise one or more of: vinylene carbonate, VC; vinyl ethylene carbonate, VEC; and fluoroethylene carbonate, FEC.
Clause 18. The aerosol provision device of any one of clauses 1 to 17, wherein the at least one supercapacitor is capable of being charged from a discharged state to a charged state in a charging time of less than or equal to 12 minutes, and in the charged state the at least one supercapacitor is configured to store sufficient electrical charge to supply electrical power to the at least one aerosol generator for no more than 200 puffs.
Clause 19. The aerosol provision device of any one of clauses 1 to 18, wherein between a first output voltage limit and a second output voltage limit, the at least one supercapacitor has a discharge capacity of no more than 450 mAh and/or has a charge capacity of no more than 400 mAh.
Clause 20. The aerosol provision device of any one of clauses 1 to 19, wherein the at least one supercapacitor is configured to be operable for a number of charge and discharge cycles greater than or equal to 10,000 cycles, and wherein after said number of charge and discharge cycles the at least one supercapacitor is still capable of storing sufficient electrical charge to supply electrical power to the at least one aerosol generator for no more than 200 puffs.
Clause 21. The aerosol provision device of any one of clauses 1 to 20, comprising:
   circuitry configured to control a supply of electrical power to the at least one supercapacitor during a charging operation, to charge the at least one supercapacitor from a discharged state to a charged state,
   wherein the circuitry is configured to control the supply of electrical power to the at least one supercapacitor during the charging operation so as to charge the at least one supercapacitor at a C-rate greater than or equal to 25C,
   wherein between a first output voltage limit and a second output voltage limit, the at least one supercapacitor has a discharge capacity of no more than 450 mAh and/or has a charge capacity of no more than 400 mAh, and/or wherein in the charged state the at least one supercapacitor is configured to store sufficient electrical charge to supply electrical power to the at least one aerosol generator for no more than 200 puffs.
Clause 22. The aerosol provision device of any one of clauses 1 to 21, wherein the at least one supercapacitor is in the form of a cell having any one of the following cell shapes: a cylindrical cell shape, a pouch cell shape, a rectangular cell shape, and a prismatic cell shape.
Clause 23. The aerosol provision device of any one of clauses 1 to 22, wherein the at least one supercapacitor is configured to be removeable and/or replaceable from the aerosol provision device.
Clause 24. An aerosol provision system comprising:
   a consumable; and
   the aerosol provision device of any one of clauses 1 to 23, wherein the aerosol provision device is configured to be releasably engageable with said consumable,
   wherein said consumable comprises the aerosol-generating material.
Clause 25. The aerosol provision system according to clause 24, wherein the consumable comprises the at least one aerosol generator.

## Claims

1. An aerosol provision device for providing an aerosol during a puff on the aerosol provision device, the aerosol provision device comprising:
at least one power source comprising at least one supercapacitor configured to supply electrical power to at least one aerosol generator for generating the aerosol from aerosol-generating material,
wherein the at least one supercapacitor is a lithium-ion capacitor, LIC, comprising an anode and a cathode, and wherein said anode and/or said cathode comprises one or more dopants.

2. The aerosol provision device of claim 1, wherein said cathode comprises one or more of: activated carbon, heteroatom-doped carbon, or graphene.

3. The aerosol provision device of claim 1 or 2, wherein said cathode comprises one or more of the following as said one or more dopants: lithium cobalt oxide, LCO; lithium manganese oxide, LMO; lithium iron phosphate, LFP; lithium iron manganese phosphate, LMFP; lithium nickel aluminium oxide, NCA; and lithium nickel manganese cobalt oxide, NMC.

4. The aerosol provision device of claim 1, 2 or 3, wherein said anode comprises one or more of: lithium pre-doped graphitic carbon; hard carbon and/or soft carbon; lithium titanium oxide, LTO; and iron oxide, Fe₂O₃.

5. The aerosol provision device of any one of claims 1 to 4, wherein said one or more dopants include one or more cation dopants and/or one or more anion dopants.

6. The aerosol provision device of claim 5, wherein said one or more cation dopants comprise one or more of: Na; K; Mg; Zn; Ca; Fe; Al; Y; Cr; Mo; Te; Zr; Si; Sn; Ti; Ru; V; W; Ta; Ga; and Nb.

7. The aerosol provision device of claim 5 or 6, wherein said one or more anion dopants comprise one or more of: F; Cl; S; N; Si; B; and P.

8. The aerosol provision device of any one of claims 1 to 7, wherein the at least one supercapacitor comprises a liquid electrolyte.

9. The aerosol provision device of claim 8, wherein the liquid electrolyte comprises one or more of the following: a lithium salt; lithium hexafluoroarsenate, LiAsF₆; lithium bis (fluorosulfonyl) imide, LiFSI; lithium bis (trifluoromethanesulfonyl) imide, LiTFSI; lithium tetrafluoroborate, LiBF₄; lithium perchlorate, LiClO₄; and lithium bis(oxalato) borate, LiBOB.

10. The aerosol provision device of claim 9, wherein the lithium salt comprises lithium hexafluorophosphate, LiPF₆.

11. The aerosol provision device of any one of claims 8 to 10, wherein the liquid electrolyte comprises a carbonate-based organic solvent or a nitrile-based organic solvent, and/or comprises one or more additives.

12. The aerosol provision device of claim 11, wherein the carbonate-based organic solvent comprises one or more of: ethylene carbonate, EC; propylene carbonate, PC; ethyl methyl carbonate, EMC; diethyl carbonate, DEC; dimethyl carbonate, DMC; propyl acetate, PA; methyl formate, MF; methyl acetate, MA; ethyl acetate, EA; methyl pyrrolidone, MP; tetrahydrofuran, THF; dimethylsulfoxide, DMSO; 1,2-dimenthoxyethane, DME; and dichloromethane, DCM.

13. The aerosol provision device of claim 11 or 12, wherein the nitrile-based organic solvent comprises acetonitrile and/or acrylonitrile.

14. The aerosol provision device of any one of claims 11 to 13, wherein said one or more additives comprise one or more of: vinylene carbonate, VC; vinyl ethylene carbonate, VEC; and fluoroethylene carbonate, FEC.

15. An aerosol provision system comprising:
a consumable; and
the aerosol provision device of any one of claims 1 to 14, wherein the aerosol provision device is configured to be releasably engageable with said consumable,
wherein said consumable comprises the aerosol-generating material.
